# EUROPEAN PATENT SPECIFICATION

(11) **EP 1 478 637 B1**
(45) Date of publication and mention of the grant of the patent: **10.08.2005**
(21) Application number: 03706736.0
(22) Date of filing: 26.02.2003
(51) Int. Cl.: C07D 307/87, A61K 31/343, A61P 25/24

(54) **AMORPHOUS CITALOPRAM SALTS**
AMORPHE CITALOPRAM SALZE
SELS AMORPHES DE CITALOPRAM

(30) Priority: 27.02.2002 GB 0204683
(43) Date of publication of application: 24.11.2004
(73) Proprietor: Cipla Ltd., Mumbai 400 008 (IN)
(72) Inventor: HAMIED, Yusuf, Khwaja, Windsor Villa, Mumbai 400 026 (IN); KANKAN, Rajendra, N., A 3/5, NBD Society, Mumbai 400 084, Maharashtra (IN); RAO, Dharmaraj, R., Thane 400 601, Maharashtra (IN)
(74) Representative: Wain, Christopher Paul
(86) International application number: PCT/GB2003/000816
(87) International publication number: WO 2003/072563

(56) References cited:
- GB-A- 1 526 331
- GB-A- 2 357 762

## Description

This invention relates to pharmaceutical salts, more particularly to citalopram salts, especially but not exclusively citalopram hydrobromide.

Citalopram is a well known antidepressant drug whose systematic name is 1-[3-(dimethylamino)propyl]-I-(4-fluorophenyl)-1,3-dihydro-5-isobenzofurancarbonitrile. It is a selective centrally acting serotonin (S-hydroxytryptamine; 5-HT) reuptake inhibitor. It is marketed as the hydrobromide or hydrochloride salt. One particular enantiomer of interest is S-citalopram and salts thereof

Citalopram was first described in GB-A-1526331 and, subsequently, a number of different processes have been described for its preparation.

The salts are prepared conventionally from the free base, in crystalline form. Purification can be effected by recrystallisation, although it is generally preferred to purify the base before conversion to the salt.

In general, the preparation of pure crystals of a citalopram salt such as the hydrobromide, is quite demanding. It can involve thin film distillation of the base, for example, or multiple crystallisation steps either of the base or of the salt.

We have now found that there are certain advantages if the salt is prepared, not in crystalline form, but in amorphous form. This is novel and constitutes one aspect of the present invention. By providing the salt in amorphous form, the problems in crystallisation of the salt (with its unavoidable product loss) are avoided.

The amorphous form of the salt can be made by conventional techniques such as lyophilisation or evaporation (preferably rotary evaporation) of a solution of the salt. We prefer, however, to use spray drying of a solution to produce the amorphous salt. This is a well known and very efficient process, involving negligible material losses.

In order to obtain pure amorphous salt (ie salt containing no more than about 1% by weight, preferably less than 0.5% by weight, impurities), the salt solution from which the amorphous salt is made must itself be pure. Pure solutions can be made by known techniques, such as by dissolving purified salt in a suitable solvent, or by dissolving purified base in a suitable solvent and then converting to the salt in solution. The pure base can be made, for example, by the known techniques such as thin film distillation (to produce an oil) or crystallisation of the base. However, in view of the disadvantages of these procedures, we prefer to purify crude citalopram base in solution by other techniques more fully described and claimed in our corresponding applications GB 0204682.9 and GB 0204680.3. Reference should be made to our said corresponding applications for further details. The citalopram base purified by these techniques is then preferably converted to a solution of the salt in order to make the salt in amorphous form.

The invention further includes pharmaceutical compositions comprising an amorphous citalopram salt of the invention and a pharmaceutically acceptable carrier. These compositions may be in any suitable form, such as tablets or capsules, as will be clear to those skilled in the art. Preferred compositions of the present invention contain amorphous S-citalopram salts, especially the hydrobromide.

In one procedure according to the invention, crystalline citalopram hydrobromide is dissolved in a solvent, such as methanol or water, and subjected to spray drying under suitable conditions to obtain amorphous citalopram hydrobromide. In another procedure, the citalopram hydrobromide is dissolved in a solvent such as methanol or water, and vacuum evaporated on a rotary evaporator, or lyophilised under suitable conditions of temperature and pressure, to obtain amorphous citalopram hydrobromide.

In a further procedure, pure citalopram base is dissolved in a suitable solvent, and a molar quantity of hydrobromic acid added such that the so-formed citalopram hydrobromide remains in solution. This solution may then be subjected to spray drying or vacuum evaporation, for example, to give amorphous citalopram hydrobromide.

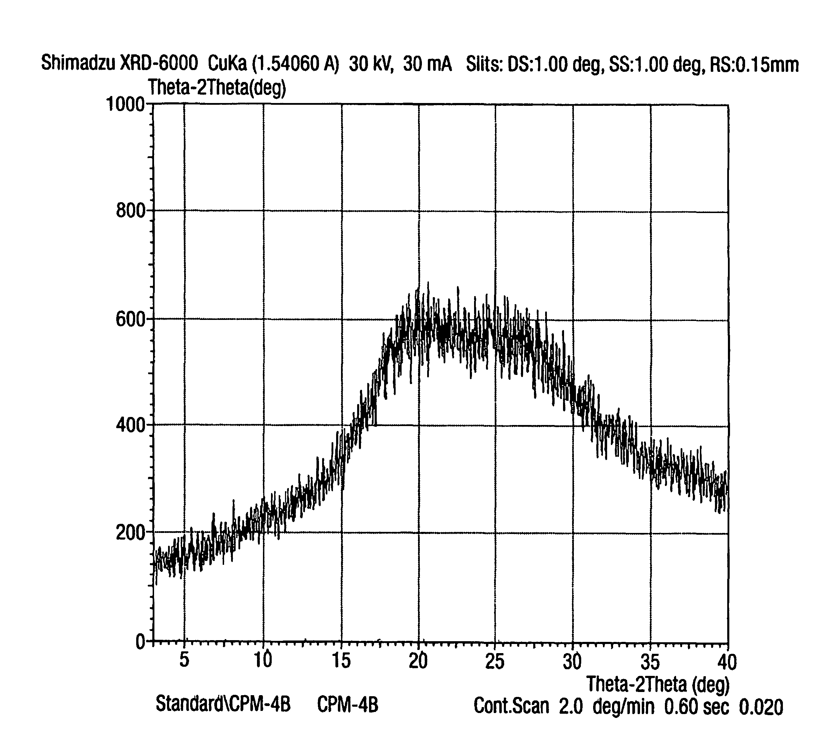

Amorphous citalopram hydrobromide according to the present invention can be characterised by powder X-ray diffraction and microscopic examination. The accompanying Figure 1 shows a typical XRD diffractogram, which is characterised by absence of well-defined peaks, which are an essential feature of a crystalline substance.

In order that the invention may be more fully understood, the following Examples are given by way of illustration only.

### Example 1

Citalopram hydrobromide 20 g was dissolved in methanol 200 ml and spray dried on Lab-Plant spray drier SD 05 with an inlet temperature of 110°C, outlet temperature of 67°C and feed rate of 5ml/min to obtain amorphous citalopram hydrobromide.

### Example 2

Citalopram hydrobromide 20 g was dissolved in 200 ml methanol and evaporated in a rotary evaporator under vacuum of 5 torr at 60°C to obtain a stable foam which solidified in about 2 hours to give amorphous citalopram hydrobromide.

### Example 3

Citalopram base 20 g was dissolved in 100 ml methanol, and 10.5 g of 48% aqueous hydrobromic acid was added to give a clear solution. This was spray dried on Lab-Plant spray drier SD 05 with an inlet temperature of 110°C, outlet temperature of 67°C and feed rate of 5ml/min to obtain amorphous citalopram hydrobromide.

### Example 4

Citalopram base 20 g was dissolved in 100 ml methanol, and 10.5 g 48% aqueous hydrobromic acid was added to give a clear solution. This solution was evaporated in a rotary-evaporator under vacuum of 5 torr at 60°C to obtain a stable foam, which solidified after 2 hours to give amorphous citalopram hydrobromide.

## Claims

1. A pharmaceutically acceptable salt of citalopram in amorphous form.

2. A pharmaceutically acceptable salt of S-citalopram in amorphous form.

3. Amorphous citalopram hydrobromide or hydrochloride.

4. A method of making a salt as claimed in claim 1, 2 or 3, which comprises spray drying a solution of the salt to convert the salt to an amorphous solid.

5. A method of making a salt as claimed in claim 1, 2 or 3, which comprises subjecting a solution of the salt to evaporation to form an amorphous solid.

6. A method according to claim 5, wherein the solution is subjected to rotary evaporation or lyophilisation.

7. A method according to claim 4, 5 or 6, wherein the salt solution is made by reacting citalopram base with an acid to form a solution of the pharmaceutically acceptable salt.

8. A pharmaceutical composition which comprises a salt as claimed in claim 1, 2 or 3, and a pharmaceutically acceptable carrier therefor.

## Patentansprüche

1. Pharmazeutisch annehmbares Salz von Citalopram in amorpher Form.

2. Pharmazeutisch annehmbares Salz von S-Citalopram in amorpher Form.

3. Amorphes Citalopram-Hydrobromid oder -Hydrochlorid.

4. Verfahren zum Herstellen eines Salzes, wie in Anspruch 1, 2 oder 3 beansprucht, welches das Sprühtrocknen einer Lösung des Salzes umfasst, um das Satz in einen amorphen Feststoff umzuwandeln.

5. Verfahren zum Herstellen eines Salzes wie in Anspruch 1, 2 oder 3 beansprucht, welches das Verdampfen einer Lösung des Salzes umfasst, um einen amorphen Feststoff zu bilden.

6. Verfahren gemäß Anspruch 5, worin die Lösung einer Rotationsverdampfung oder Gefriertrocknung unterworfen wird.

7. Verfahren gemäß Anspruch 4, 5 oder 6, worin die Salzlösung hergestellt wird, indem Citaloprambase mit einer Säure umgesetzt wird, um eine Lösung des pharmazeutisch annehmbaren Salzes zu bilden.

8. Pharmazeutische Zusammensetzung, welche ein Salz, wie in Anspruch 1, 2 oder 3 beansprucht, und einen pharmazeutisch annehmbaren Träger dafür umfasst.

## Revendications

1. Sel de citalopram pharmaceutiquement acceptable sous forme amorphe.

2. Sel de S-citalopram pharmaceutiquement acceptable sous forme amorphe.

3. Chlorhydrate ou bromhydrate de citalopram amorphe.

4. Procédé pour la préparation d'un sel selon l'une des revendications 1, 2 ou 3, qui comprend le séchage par pulvérisation d'une solution du sel pour convertir le sel en un solide amorphe.

5. Procédé pour la préparation d'un sel selon l'une des revendications 1, 2 ou 3, qui comprend la soumission d'une solution du sel à une évaporation pour former un solide amorphe.

6. Procédé selon la revendication 5, dans lequel la solution est soumise à une évaporation rotative ou à une lyophilisation.

7. Procédé selon l'une des revendications 4, 5 ou 6, dans lequel la solution de sel est préparée en faisant réagir le citalopram avec un acide pour former une solution du sel pharmaceutiquement acceptable.

8. Composition pharmaceutique qui comprend un sel selon l'une des revendications 1, 2 ou 3 et pharmaceutiquement acceptable.
